# EUROPÄISCHE PATENTSCHRIFT

(11) **EP 3 191 446 B1**
(45) Veröffentlichungstag und Bekanntmachung des Hinweises auf die Patenterteilung: **25.08.2021**
(21) Anmeldenummer: 15759769.1
(22) Anmeldetag: 04.09.2015
(51) Int. Cl.: C07C 67/20, C01C 3/02, B01J 20/20

(54) **VERFAHREN ZUR HERSTELLUNG VON ALPHA-HYDROXYCARBONSÄUREESTERN UNTER REZYKLIERUNG VON AMMONIAK**
METHOD FOR THE PREPARATION OF ALPHA-HYDROXY CARBOXYLIC ACID ESTERS FROM THE RECYCLING OF AMMONIA
PROCÉDÉ DE FABRICATION D'ESTERS D'ACIDE ALPHA-HYDROXYDARBOXYLIQUE DANS LE RECYCLAGE D'AMMONIAC

(30) Priorität: 10.09.2014 EP 14184249
(43) Veröffentlichungstag der Anmeldung: 19.07.2017
(73) Patentinhaber: Röhm GmbH, 64295 Darmstadt (DE)
(72) Erfinder: KRILL, Steffen, 64367 Mühltal (DE); AIT AISSA, Belaid, 64287 Darmstadt (DE); MAY, Alexander, 64342 Seeheim-Jugenheim (DE); TRESKOW, Marcel, 64293 Darmstadt (DE)
(74) Vertreter: Röhm Patent Association
(86) Internationale Anmeldenummer: PCT/EP2015/070196
(87) Internationale Veröffentlichungsnummer: WO 2016/037928

(56) Entgegenhaltungen:
- EP-A1- 0 922 674
- EP-A2- 0 941 984
- WO-A1-2008/009503
- None

## Beschreibung

Die vorliegende Erfindung beschreibt ein Verfahren zur Herstellung von alpha-Hydroxycarbonsäureestern (HCSE) ausgehend von Blausäure, wobei das im Alkoholyseschritt des entsprechenden alpha-Hydroxycarbonsäuresamids (HCA) entstehende Ammoniak nach einem Reinigungsschritt in ein Blausäureherstellverfahren rezykliert wird.

Die Herstellung von HSCE ist aus dem Stand der Technik (WO 2008/009503) hinreichend bekannt. Insbesondere beschreiben die Anmeldungen EP 2018362 und WO 2013/026603 entsprechende Verfahren, wobei die Alkoholyse unter Druck in flüssiger Phase erfolgt und entstehendes Ammoniak bei Drücken größer 1 bar abdestilliert, bzw. der entstehende HCSE über die Gasphase abgetrennt wird.

Das gravierende Problem dieses Stands der Technik bzw. auch bei bekannten Verfahren, bei denen genannte Alkoholyse in der Gasphase erfolgt, besteht jedoch darin, dass das isolierte Ammoniak beim Rezyklieren in den Blausäureherstellprozess bereits nach kurzer Zeit zu erheblichen Aktivitätsverlusten am Katalysator führt und einen wirtschaftlichen Betrieb der Anlage unmöglich macht.

Aufgabe der vorliegenden Erfindung ist es daher, das aus der Herstellung von HCSE ausgehend von Blausäure anfallende Ammoniak so aufzubereiten, dass es problemlos, d.h. ohne Einbußen in Reaktionszeit, Ausbeute und Qualität, in ein Blausäureherstellverfahren rezykliert werden kann.

Überraschend gelöst werden diese und weitere, nicht explizit genannte Aufgaben durch die erfindungsgemäße Bereitstellung eines Verfahrens zur Herstellung von HCSE ausgehend von Blausäure dadurch gekennzeichnet, dass im Alkoholyseschritt des entsprechenden alpha-Hydroxycarbonsäureamids entstehendes Ammoniak nach einem Reinigungsschritt mit Aktivkohle noch enthaltend mindestens ein Alkylamin in den Blausäureherstellprozess (HCN-Prozess) rezykliert wird. Überraschend ist diese Lösung insofern, als durch den erfindungsgemäßen Reinigungsschritt nicht alle Verunreinigungen des aus der Alkoholysereaktion entstehenden Ammoniaks, insbesondere Alkylamine, entfernt werden und dennoch ein problemloses Betreiben eines HCN-Prozesses möglich ist.

Zu den im erfindungsgemäßen Verfahren einsetzbaren alpha-Hydroxycarbonsäureamiden gehören üblicherweise all diejenigen Carbonsäureamide, die in alpha-Stellung zur Carbonsäureamidgruppe wenigstens eine Hydroxygruppe aufweisen.

Carbonsäureamide wiederum sind in der Fachwelt allgemein bekannt. Üblicherweise werden hierunter Verbindungen mit Gruppen der Formel -CONR'R"- , worin R' und R" unabhängig Wasserstoff oder eine 1-30 Kohlenstoffatome aufweisende Gruppe darstellt, die insbesondere 1-20, bevorzugt 1-10 und insbesondere 1-5 Kohlenstoffatome umfasst. Das Carbonsäureamid kann 1, 2, 3, 4 oder mehr Gruppen der Formel -CONR'R"- umfassen. Hierzu gehören insbesondere Verbindungen der Formel R(-CONR'R")ₙ, worin der Rest R eine 1-30 Kohlenstoffatome aufweisende Gruppe darstellt, die insbesondere 1-20, bevorzugt 1-10, insbesondere 1-5 und besonders bevorzugt 2-3 Kohlenstoffatome umfasst, R' und R" die zuvor genannte Bedeutung hat und n eine ganze Zahl im Bereich von 1-10, vorzugsweise 1-4 und besonders bevorzugt 1 oder 2 darstellt.

Der Ausdruck "1 bis 30 Kohlenstoffatome aufweisende Gruppe" kennzeichnet Reste organischer Verbindungen mit 1 bis 30 Kohlenstoffatomen. Er umfasst neben aromatischen und heteroaromatischen Gruppen auch aliphatische und heteroaliphatische Gruppen, wie beispielsweise Alkyl-, Cycloalkyl-, Alkoxy-, Cycloalkoxy-, Cycloalkylthio- und Alkenylgruppen. Dabei können die genannten Gruppen verzweigt oder nicht verzweigt sein.

Erfindungsgemäß bezeichnen aromatische Gruppen Reste ein- oder mehrkerniger aromatischer Verbindungen mit vorzugsweise 6 bis 20, insbesondere 6 bis 12 C-Atomen. Heteroaromatische Gruppen kennzeichnen Arylreste, worin mindestens eine CH-Gruppe durch N ersetzt ist und/oder mindestens zwei benachbarte CH-Gruppen durch S, NH oder O ersetzt sind.

Erfindungsgemäß bevorzugte aromatische oder heteroaromatische Gruppen leiten sich von Benzol, Naphthalin, Biphenyl, Diphenylether, Diphenylmethan, Diphenyldimethylmethan, Bisphenon, Diphenylsulfon, Thiophen, Furan, Pyrrol, Thiazol, Oxazol, Imidazol, Isothiazol, Isoxazol, Pyrazol, 1,3,4-Oxadiazol, 2,5-Diphenyl-1,3,4-oxadiazol, 1,3,4-Thiadiazol, 1,3,4-Triazol, 2,5-Diphenyl-1,3,4-triazol, 1,2,5-Triphenyl-1,3,4-triazol, 1,2,4-Oxadiazol, 1,2,4-Thiadiazol, 1,2,4-Triazol, 1,2,3-Triazol, 1,2,3,4-Tetrazol, Benzo[b]thiophen, Benzo[b]furan, Indol, Benzo[c]thiophen, Benzo[c]furan, Isoindol, Benzoxazol, Benzothiazol, Benzimidazol, Benzisoxazol, Benzisothiazol, Benzopyrazol, Benzothiadiazol, Benzotriazol, Dibenzofuran, Dibenzothiophen, Carbazol, Pyridin, Bipyridin, Pyrazin, Pyrazol, Pyrimidin, Pyridazin, 1,3,5-Triazin, 1,2,4-Triazin, 1,2,4,5-Triazin, Tetrazin, Chinolin, Isochinolin, Chinoxalin, Chinazolin, Cinnolin, 1,8-Naphthyridin, 1,5-Naphthyridin, 1,6-Naphthyridin, 1,7-Naphthyridin, Phthalazin, Pyridopyrimidin, Purin, Pteridin oder Chinolizin, 4H-Chinolizin, Diphenylether, Anthracen, Benzopyrrol, Benzooxathiadiazol, Benzooxadiazol, Benzopyridin, Benzopyrazin, Benzopyrazidin, Benzopyrimidin, Benzotriazin, Indolizin, Pyridopyridin, Imidazopyrimidin, Pyrazinopyrimidin, Carbazol, Aciridin, Phenazin, Benzochinolin, Phenoxazin, Phenothiazin, Acridizin, Benzopteridin, Phenanthrolin und Phenanthren ab, die gegebenenfalls auch substituiert sein können.

Zu den bevorzugten Alkylgruppen gehören die Methyl-, Ethyl-, Propyl-, Isopropyl-, 1-Butyl-, 2-Butyl-, 2-Methylpropyl-, tert.-Butyl-, Pentyl-, 2-Methylbutyl-, 1,1-Dimethylpropyl-, Hexyl-, Heptyl-, Octyl-, 1,1,3,3-Tetramethylbutyl, Nonyl-, 1-Decyl-, 2-Decyl-, Undecyl-, Dodecyl-, Pentadecyl- und die Eicosyl-Gruppe.

Zu den bevorzugten Cycloalkylgruppen gehören die Cyclopropyl-, Cyclobutyl-, Cyclopentyl-, Cyclohexyl-, Cycloheptyl- und die Cyclooctyl-Gruppe, die gegebenenfalls mit verzweigten oder nicht verzweigten Alkylgruppen substituiert sind.

Zu den bevorzugten Alkenylgruppen gehören die Vinyl-, Allyl-, 2-Methyl-2-propen-, 2-Butenyl-, 2-Pentenyl-, 2-Decenyl- und die 2-Eicosenyl-Gruppe.

Zu den bevorzugten heteroaliphatischen Gruppen gehören die vorstehend genannten bevorzugten Alkyl- und Cycloalkylreste, in denen mindestens eine Kohlenstoff-Einheit durch O, S oder eine Gruppe NR⁸ oder NR⁸R⁹ ersetzt ist und R⁸ und R⁹ unabhängig eine 1 bis 6 Kohlenstoffatome aufweisende Alkyl-, eine 1 bis 6 Kohlenstoffatome aufweisende Alkoxy- oder eine Arylgruppe bedeutet.

Erfindungsgemäß ganz besonders bevorzugt weisen die Carbonsäureamide verzweigte oder nicht verzweigte Alkyl-, oder Alkoxygruppen mit 1 bis 20 Kohlenstoffatomen, vorzugsweise 1 bis 12, zweckmäßigerweise 1 bis 6, insbesondere 1 bis 4 Kohlenstoffatomen und Cycloalkyl- bzw. Cycloalkyloxygruppen mit 3 bis 20 Kohlenstoffatomen, vorzugsweise 5 bis 6 Kohlenstoffatomen auf.

Der Rest R kann Substituenten aufweisen. Zu den bevorzugten Substituenten gehören u.a. Halogene, insbesondere Fluor, Chlor, Brom, sowie Alkoxy- oder Hydroxyreste.

Die alpha-Hydroxycarbonsäureamide können beim Verfahren der Erfindung einzeln oder als Mischung von zwei oder drei oder mehreren unterschiedlichen alpha-Hydroxycarbonsäureamiden eingesetzt werden. Zu besonders bevorzugten alpha-Hydroxycarbonsäureamiden gehören alpha-Hydroxyisobuttersäureamid (HIBA) und/oder alpha-Hydroxypropionsäureamid.

Weiterhin ist es in einer Abwandlung des erfindungsgemäßen Verfahrens von besonderem Interesse solche alpha-Hydroxycarbonsäureamide einzusetzen, die durch Cyanhydrinsynthese aus Ketonen oder Aldehyden und Blausäure zugänglich sind. In einem ersten Schritt wird hierbei die Carbonylverbindung, beispielsweise ein Keton, insbesondere Aceton, oder ein Aldehyd, beispielsweise Acetaldehyd, Propanal, Butanal, mit Blausäure zum jeweiligen Cyanhydrin umgesetzt. Besonders bevorzugt wird hierbei Aceton und/oder Acetaldehyd auf typische Weise unter Verwendung einer geringen Menge an Alkali oder eines Amins als Katalysator umgesetzt. In einem weiteren Schritt wird das so erhaltene Cyanhydrin mit Wasser zum alpha-Hydroxycarbonsäureamid umgesetzt.

Zu den bei Verfahren der Erfindung mit Erfolg einsetzbaren Alkoholen gehören alle dem Fachmann geläufigen Alkohole sowie Vorläuferverbindungen von Alkoholen, die unter den angegebenen Bedingungen von Druck und Temperatur in der Lage sind, mit den HCA im Sinne einer Alkoholyse zu reagieren. Bevorzugt erfolgt die Umsetzung des HCA durch Alkoholyse mit einem Alkohol, der vorzugsweise 1-10 Kohlenstoffatome, besonders bevorzugt 1 bis 5 Kohlenstoffatome umfasst. Bevorzugte Alkohole sind u.a. Methanol, Ethanol, Propanol, Butanol, insbesondere n-Butanol und 2-Methyl-1-propanol, Pentanol, Hexanol, Heptanol, 2-Ethylhexanol, Octanol, Nonanol und Decanol und deren Mischungen. Besonders bevorzugt wird als Alkohol Methanol und / oder Ethanol eingesetzt, wobei Methanol ganz besonders zweckmäßig ist. Auch der Einsatz von Vorstufen eines Alkohols ist prinzipiell möglich. So können beispielsweise Alkylformiate eingesetzt werden. Insbesondere eignen sich Methylformiat oder eine Mischung von Methanol und Kohlenmonoxid.

Im Rahmen der Erfindung hat es sich herausgestellt, dass die geschilderte Vorgehensweise ein breites Spektrum an Mengenverhältnissen der Edukte tolerieren kann. So kann man die Alkoholyse bei einem relativ großen Alkoholüberschuss oder -unterschuss im Verhältnis zum HCA durchführen. Besonders bevorzugt sind Verfahrensvariaten, bei denen man die Umsetzung der Edukte bei einem molaren Ausgangsverhältnis von Alkohol zu HCA im Bereich von 1:3 bis 20:1 vornimmt. Ganz besonders zweckmäßig ist das Verhältnis 1:2 bis 15:1 und noch zweckmäßiger 1:1 bis 10:1.

In einer Ausführungsform des erfindungsgemäßen Verfahrens wird die Umsetzung zwischen alpha-Hydroxycarbonsäureamid und Alkohol in einem Druckreaktor durchgeführt. Hierzu werden EP 2018362 und WO 2013/026603 zu Offenbarungszwecken durch Referenz in die vorliegende Anmeldung eingefügt.

Die Alkoholyse wird unter einem Druck von 1-100 bar durchgeführt. Ferner ist auch während der Abtrennung/Entfernung des Ammoniaks aus dem Produktgemisch der Druck größer als 1 bar. Insbesondere bedeutet dies, dass auch das bei der Umsetzung entstehende Ammoniak aus der Mischung unter einem Druck von größer 1 bar abdestilliert wird, wobei auf den Einsatz von Hilfsmitteln wie Strip-Gas zur destillativen Entfernung des Ammoniaks vollständig verzichtet wird.

Die Produktmischung wird im Sinne der Erfindung nicht nur an Ammoniak abgereichert sondern auch an nicht umgesetztem Alkohol. Gerade für den Fall, dass man zur Alkoholyse Methanol verwendet, resultiert ein Produktgemisch unter anderem mit den prinzipiell sehr schwer voneinander trennbaren Komponenten Ammoniak und Methanol. Im einfachsten Fall entfernt man zur Abreicherung des Produktgemisches an Ammoniak und Alkohol die beiden Komponenten direkt als Stoffgemisch aus dem Produktgemisch. Die beiden Stoffe werden dann einer nachgeschalteten Trennoperation, zum Beispiel einer Rektifikation, unterworfen. Andererseits ist es im Sinne der Erfindung auch möglich, die beiden Komponenten Alkohol (Methanol) und Ammoniak in einem Vorgang vom Produktgemisch abzutrennen und dabei die beiden Bestandteile Ammoniak und Alkohol (Methanol) auch noch voneinander zu separieren.

In einer bevorzugten Verfahrensabwandlung der Erfindung kann es von besonderem Interesse sein, dass man den Umsetzungsschritt und die Entfernung des Ammoniaks/Alkohols aus der Produktmischung räumlich voneinander trennt und in unterschiedlichen Aggregaten durchführt. Zu diesem Zweck kann man beispielsweise ein oder mehrere Druckreaktoren vorsehen und diese mit einer Druckdestillationskolonne verbinden. Hierbei handelt es sich um einen oder mehrere Reaktoren, die außerhalb der Kolonne in einem separaten Bereich angeordnet sind.

Im weitesten Sinne beinhaltet dies, dass man Eduktstöme umfassend als Edukte ein alpha-Hydroxycarbonsäureamid, einen Alkohol in einen Druckreaktor einspeist, die Eduktströme im Druckreaktor bei einem Druck im Bereich von 1-100 bar katalytisch miteinander umsetzt, die resultierende Produktmischung aus dem Druckreaktor ausschleust und die Produktmischung an Alkohol und Ammoniak abreichert, wobei Ammoniak bei einem Druck, der ständig größer als 1 bar gehalten wird, abdestilliert wird.

In einer besonderen Ausführungsform dieser Verfahrensvariante findet die Umsetzung der Edukte und Abtrennung von Ammoniak/Alkohol in zwei verschiedenen räumlich voneinander getrennten Aggregaten statt. Dies hat den Vorteil, dass man für die Reaktion/Umsetzung der Edukte und die anschließende Abtrennung von Ammoniak/Alkohol unterschiedliche Druckbereiche anwenden kann. Durch die Auftrennung des Verfahrens in einen Umsetzungsschritt im Druckreaktor unter höherem Druck als in einem Trennschritt in einer Druckkolonne, wobei beide Schritte unter Überdruck, d.h. größer als 1 bar geführt werden, gelingt es, die Trennwirkung nochmals signifikant zu verbessern und die Effizienz der Abtrennung des Ammoniak/Alkohol-Gemisches zu vergrößern.

Die genannten Qualitätsmerkmale lassen sich noch weiter verbessern, indem man die Reaktion im Druckreaktor einmal oder mehrmals mit der Richtung Sumpf der Trennkolonne (Druckdestillationskolonne) an Ammoniak und Alkohol abgereicherten Produktmischung wiederholt, wobei der Umsetzungsschritt auf eine Mehrzahl von Druckreaktoren verlagert wird, die in Reihe geschaltet sind.

Für die angegebene Verfahrensvariante haben sich verschiedene Temperaturbereiche in Kolonne und Reaktor als zweckmäßig erwiesen. So weist die Druckdestillationskolonne im Allgemeinen und bevorzugt eine Temperatur im Bereich von etwa 50 °C bis etwa 180 °C auf. Die exakte Temperatur wird typisch durch das siedende System in Abhängigkeit der herrschenden Druckbedingungen eingestellt. Die Temperatur im Reaktor bei der Umsetzung von HIBA mit Methanol liegt bevorzugt im Bereich von etwa 120-240 °C.

Neben der beschriebenen Variante, bei welcher die Umsetzung des alpha-Hydroxycarbonsäureamids mit dem Alkohol von der Entfernung des dabei unter anderem resultierenden Ammoniaks in zwei räumlich voneinander getrennten aber verbundenen Aggregaten durchgeführt wird, kann es in einer weiteren Verfahrensmodifikation bevorzugt sein, den Umsetzungsschritt und den Entfernungsschritt in einem einzigen Aggregat vorzunehmen. Druckreaktor und Druckdestillationskolonne werden dabei in einem einzigen Aggregat verwirklicht, fallen quasi zusammen.

In einer weiteren Variante des erfindungsgemäßen Verfahrens wird der erhaltene HCSE zumindest teilweise, bevorzugt mindestens 60 Gew%, aus der Reaktionsmischung über die Gasphase abgetrennt.

Dementsprechend wird diese Variante vorzugsweise dergestalt ausgeführt, dass ein möglichst hoher Anteil des Produkts in die Gasphase überführt wird. Dieses Ziel kann insbesondere durch die Auswahl des Reaktors, durch die Wahl des Drucks und der Temperatur sowie das Gasvolumen beim Betreiben des Reaktors, insbesondere in Bezug desselben auf das Gesamtvolumen bzw. des Flüssigkeitsvolumens erreicht werden.

Hierbei kann die Reaktion so ausgeführt werden, dass der HCSE in einem separaten Schritt von der freigesetzten stickstoffhaltigen Verbindung aus der Reaktionsmischung abgetrennt wird. Vorteile ergeben sich bei Ausführungsformen, die dadurch gekennzeichnet sind, dass der HCSE vorzugsweise zusammen mit dem freigesetzten Ammoniak aus der Reaktionsmischung abgetrennt wird.

Weitere Vorteile ergeben sich insbesondere durch Verfahren bei denen das molare Verhältnis von HCSE zu Ammoniak während der Abtrennung dieser Komponenten aus der Reaktionsmischung im Bereich von 2:1 bis 1:2 liegt. Von Interesse sind insbesondere Verfahren, bei denen die Konzentration an HCSE in der flüssigen Phase der Reaktionsmischung vorzugsweise kleiner als 30 Gew%, gehalten wird. Vorzugsweise ist das molare Verhältnis von HCSE zu alpha-Hydroxycarbonsäureamid in der flüssigen Phase der Reaktionsmischung kleiner als 1.

Zusätzliche Vorteile hinsichtlich der Produktivität des Verfahrens können dadurch erzielt werden, dass der Alkohol als Gas in die Reaktionsmischung eingeleitet wird. Die Art des Reaktors zur Durchführung des vorliegenden Verfahrens ist nicht begrenzt. Vorzugsweise werden jedoch solche Reaktoren eingesetzt, in die größere Gasmengen ein- oder ausgeleitet werden können. Bevorzugt werden dementsprechend Multiphasenreaktoren zur Durchführung dieser Verfahrensvariante eingesetzt. Hierbei können Multiphasenreaktoren eingesetzt werden, bei denen ein Gas im Gegenstrom bezüglich der Flüssigkeitsphase eingeleitet wird. Zu diesen Reaktoren gehören unter anderem Reaktoren, die auf begasten Rührkesseln oder Kaskaden beruhen. Weiterhin kann der Alkohol als Gas im Gegenstrom zu der Flüssigkeit durch eine Bodenkolonne oder Füllkörperkolonne geleitet werden.

Gemäß einer bevorzugten Ausführungsform kann der Alkohol im Gleichstrom in die Reaktionsmischung eingeleitet werden. Besonders geeignete Reaktoren dazu sind unter anderem Rieselbettreaktoren, Blasensäulenreaktoren, Strahlwäscher und Fallfilmreaktoren, wobei Rieselbettreaktoren und Fallfilmreaktoren bzw. die Kombination aus Rieselbettreaktoren und Fallfilmreaktoren besonders bevorzugt sind.

Die Umsetzung gemäß der Erfindung findet in Gegenwart eines Katalysators statt. Diese umfassen homogene Katalysatoren sowie heterogene Katalysatoren.

Beispielhafte homogene Katalysatoren für die Durchführung des erfindungsgemäßen Verfahrens sind wasserbeständige Lanthanoidverbindungen. Lanthanoidverbindungen bezeichnen Verbindungen aus der Gruppe der Lanthanoide, wie La, Ce, Pr, Nd, Pm, Sm, Eu, Gd, Td, Dy, Ho, Er, Tm, Yb und/oder Lu. Bevorzugt wird eine Lanthanoidverbindung eingesetzt, die Lanthan umfasst.

Vorzugsweise weist die Lanthanoidverbindung eine Löslichkeit in Wasser von mindestens 1 g/l, bevorzugt mindestens 10 g/l bei 25 °C auf.

Bevorzugte Lanthanoidverbindungen stellen Salze dar, die vorzugsweise in der Oxidationsstufe 3 vorliegen.

Neben den bevorzugten Varianten der homogenen Katalyse sind auch Verfahren unter Anwendung heterogener Katalysatoren zweckmäßig. Zu den mit Erfolg anwendbaren heterogenen Katalysatoren gehören unter anderem Magnesiumoxid, Calciumoxid sowie basische Ionenaustauscher und dergleichen mehr.

So können beispielsweise Verfahren bevorzugt sein, bei denen der Katalysator ein unlösliches Metalloxid ist, welches mindestens ein aus der aus Sb, Sc, V, La, Ce, Ti, Zr, Hf, V, Nb, Ta, Cr, Mo, W, Tc, Re, Fe, Co, Ni, Cu, Al, Si, Sn, Pb und Bi bestehenden Gruppe ausgewähltes Element enthält.

Alternativ dazu können Verfahren bevorzugt sein, wobei man als Katalysator ein unlösliches Metall einsetzt, ausgewählt aus der Gruppe bestehend aus Ti, Zr, Hf, V, Nb, Ta, Cr, Mo, W, Fe, Co, Ni, Cu, Ga, In, Bi und Te.

Zu den bevorzugten heterogenen Katalysatoren gehören insbesondere Katalysatoren auf Basis von ZrO₂ und/oder Al₂O₃. Besonders bevorzugte Katalysatoren dieser Gattung werden insbesondere in JP 6-345692 ausführlich dargestellt, wobei die in der Druckschrift JP 06-345692 dargelegten Katalysatoren zu Offenbarungszwecken durch Referenz in die vorliegende Anmeldung eingefügt werden.

Weitere geeignete Katalysatoren sind in der DE 102013213699, angemeldet am DPMA am 12.07.2013, beschrieben. Diese Druckschrift wird zu Offenbarungszwecken durch Referenz in die vorliegende Anmeldung eingefügt.

Besonders bevorzugt sind darin Katalysatoren auf der Basis von ZrO₂ und Al₂O₃, wobei ganz besonders bevorzugt Lanthanoxid-, Siliciumoxid- oder Yttriumoxiddotierte ZrO₂-Katalysatoren zum Einsatz kommen. Letztere sind kommerziell z.B. als Zirkonoxid-Katalysator SZ 61157 der Firma Saint-Gobain Nopro erhältlich. Das im Zirkonoxid-Kristallgitter eingebaute Yttrium bewirkt eine Stabilisierung der sonst nur oberhalb von 1200 °C stabilen tetragonalen Phase des Zirkonoxids auch bei Raumtemperatur. In der Technik werden sie als Sauerstoffleiter für Festoxid-Brennstoffzellen oder in Sauerstoff-Messgeräten (λ-Sonde) eingesetzt. Eine Zusammensetzung mit 8 mol% Y₂O₃ ist hier typisch. Im erfindungsgemäßen Verfahren werden Lanthanoxid-, Siliciumoxid- oder Yttriumoxid--Gehalte bezogen auf ZrO₂ von 0,05-20 mol%, bevorzugt von 0,5-15 mol%, besonders bevorzugt 1-10 mol% und ganz besonders bevorzugt von 2-5 mol% eingesetzt. Es können auch Mischungen der genannten Katalysatoren eingesetzt werden.

Beim Einsatz von Al₂O₃ hat sich eine Dotierung mit BaO bewährt. Gute Ergebnisse werden mit 0,01-1,2 mol% BaO bezogen auf Al₂O₃ erzielt. Besonders bevorzugt sind 0,05-1,0 mol%, ganz besonders bevorzugt 0,1-0,8 mol%.

Überraschender Weise wurde gefunden, dass diese Katalysatoren eine hohe Toleranz gegenüber der Gegenwart von Wasser aufweisen. So kann in der Alkoholyse-Reaktion der Wassergehalt im Eduktfeed 0,1-20 mol% betragen. Bevorzugt sind 0,5-10 mol%, besonders bevorzugt 1-3 mol%.

Die Reaktionstemperatur kann über einen weiten Bereich variieren, wobei die Reaktionsgeschwindigkeit mit zunehmender Temperatur im Allgemeinen zunimmt. Die obere Temperaturgrenze ergibt sich im Allgemeinen aus dem Siedepunkt des eingesetzten Alkohols. Vorzugsweise liegt die Reaktionstemperatur im Bereich von 40-300 °C, besonders bevorzugt von 120-240 °C.

In einer weiteren Variante des erfindungsgemäßen Verfahrens kann die Alkoholyse in der Gasphase erfolgen. Als beispielhafte, das erfindungsgemäße Verfahren nicht limitierende Gasphasen-Variante wird hiermit die EP 2415750 offenbart.

Dort wird ein Gasphasenverfahren beschrieben, das in Gegenwart eines Zirkondioxid-Katalysators bei Temperaturen von 150-270 °C und Drücken von 1-300 kPa, wobei der Zirkondioxid-Katalysator auch Elemente wie B, AL, Mn, Co, Ni, Y, La oder Yb oder deren Mischungen enthalten kann.

Dieses Gasphasenverfahren wird mit den genannten heterogenen Katalysatoren in einem Fest- oder Wirbelbett-Reaktor durchgeführt. Die Reaktion verläuft hierbei prinzipiell in der Gasphase, wobei der Anteil der Flüssigphase 10 Gew% oder weniger beträgt, bezogen auf die Gesamtmenge der Einsatzstoffe.

Alkohol und HCA können dabei vor Dosierung in den Reaktor verdampft oder im Reaktor selbst verdampft werden. Ferner können Alkohol und HCA getrennt oder bereits gemischt dem Reaktor zugeführt werden. Bevorzugt ist eine Variante, wobei die Reaktion unter Inertgas, bevorzugt Stickstoff verläuft, was aufgrund des verringerten Partialdrucks der Reaktionskomponenten eine leichtere Verdampfung erlaubt.

Wenn die Verdampfung der Reaktionskomponenten im Reaktor erfolgt, können diese zusammen mit einem Lösemittel in den Reaktor dosiert werden. Mögliche Lösemittel sind z.B. ether-basierende Lösemittel wie Tetrahydrofuran, amid-basierende wie N-Methylpyrrolidon oder ester-basierende wie Methyllactat oder ähnliche. Bevorzugt jedoch ist für diese Variante eine lösemittelfreie Ausführung der Gasphasenreaktion.

Die Reaktionstemperatur wird entsprechend so gewählt, das die Reaktionskomponenten ausreichend verdampft im Reaktor vorliegen. Diese ist abhängig von der Art des Amids bzw. des Alkohols, deren molarem Verhältnis, sowie der Anwesenheit eines Inert-Gases oder Lösemittels. Zur ausreichenden Verdampfung von HIBA wird eine Reaktionstemperatur >150 °C gewählt, bei Reaktion unter Atmosphärendruck eine >180 °C. Wird die Reaktionstemperatur <240 °C gehalten, wird die Zersetzung von HIBA zu Aceton, bzw. die Bildung von Nebenprodukten wie alpha-Alkoxyisobuttersäure oder durch Dehydrierung entstehende Olefin-Derivate vermieden.

Zur Erhaltung einer langfristig stabilen Umsetzungsrate liegt die Dosierrate bei 0,01-5 Gewichtsanteilen/h HCA bezogen auf die eingesetzte Katalysatormenge. Bevorzugt liegt die WHSV (weight hourly space velocity) bezogen auf die Reaktionskomponente Alkohol bei 0,01-100 h⁻¹.

Zur Ausschleusung des gewünschten HCSE, sowie dessen Trennung von Ammoniak, entstandenen Nebenprodukten bzw. nicht umgesetzten Ausgangsprodukten können in dieser Verfahrensvariante gängige Trennverfahren wie z.B. Destillation zum Einsatz kommen.

Eine weitere erfindungsgemäße Gasphasenverfahren-Variante wird in Gegenwart von Wasser durchgeführt. Überraschend konnte festgestellt werden, dass z.B. bei der Umsetzung von HIBA mit Methanol in Gegenwart von Wasser die Bildung von Nebenprodukten, insbesondere Aceton oder 2-Amino-2-methylpropionitril (AMPN), sehr stark unterbunden, sowie die Selektivität zu HIBSM und die Katalysatorstandzeit wesentlich erhöht werden. Wasser kann sowohl dem Edukt-Feed zugesetzt als auch direkt in den Reaktor eingespeist werden. Das molare Verhältnis von Wasser zu HCA beträgt 0,1-10, bevorzugt 0,3-5, besonders bevorzugt 0,5-1 mol/mol.

Das molare Verhältnis von Alkohol zu HCA bei dieser Variante beträgt 1-25, bevorzugt 3-20, besonders bevorzugt 5-9 mol/mol.

Bei der Alkoholyse des HCA entstehen zahlreiche Nebenprodukte, insbesondere Alkylamine und Olefine, die vom Reaktionsprodukt Ammoniak schwer zu trennen sind. Erfolgt die Alkoholyse in der bevorzugten Variante mit den Reaktionskomponenten HIBA und Methanol, entstehen bei dieser Methanolyse als Nebenprodukte Dimethoxypropan, Methoxypropen, Methylformiat, Methylacetat, Dimethyl-isopropylamin, Propylen und inbesondere Dimethylamin sowie Trimethylamin.

Wird dieses Reaktions-Ammoniak nach der Trennung vom nicht umgesetzten Alkohol als Ausgangsprodukt direkt in einen HCN-Prozess, z.B. in ein Andrussow-Verfahren, eingespeist, erfolgt bereits nach kürzester Zeit, innerhalb weniger Minuten, ein signifikanter Abfall der Katalysatoraktivität, bemerkbar an einem deutlichen Anstieg der Temperatur am Katalysator-Netz.

Überraschenderweise wurde nun gefunden, dass bei Zwischenschaltung eines Reinigungsschrittes, der Aktivkohle beinhaltet, dieser Abfall der Katalysatoraktivität vermieden werden kann, obwohl zumindest noch ein Alkylamin im, in den HCN-Prozess eingespeisten Ammoniak enthalten ist. Dabei kann die Konzentration der Alkylamin-Verunreinigung bezogen auf Ammoniak 0,1-10, bevorzugt 0,2-8, und besonders bevorzugt 0,5-6 Gew% betragen.

Aktivkohle kann in allen möglichen morphologischen Formen, als Pulver, granuliert, oder als zylindrische bzw. sphärische Pellets eingesetzt werden. Bevorzugt sind granulierte und pelletierte Aktivkohlen mit Oberflächenwerten von 1000-1500 m²/g, besonders bevorzugt 1200-1400 m²/g. Neben chemisch mit Zinkchlorid bzw. Phosphorsäure aktivierter Aktivkohle werden unter Verwendung von Alkalisalzen, Alkalimetallen, Chloriden, Sulfaten und Acetaten gas-aktivierte Aktivkohlen bevorzugt.

Besonders geeignete Aktivkohlen sind die bei Firma Donau Carbon kommerziell erhältlichen Hydraffin CC 12x40, Alcarbon DC 60/8x16 oder Supersorbon C IV.

Als erfindungsgemäße Adsorber sind Festbett-, Bewegtbett- oder Wirbelbettadsorber möglich, wobei erstere bevorzugt werden. Beispielhafte apparative Lösungen werden z.B. in Ullmann's Encyclopedia of Industrial Chemistry, Wiley 2012, S. 555 ff (DOI: 10.1002/14356007.b03_09.pub2) beschrieben. Die Verfahrensweise kann kontinuierlich oder batchweise erfolgen, ersteres ist bevorzugt.

Die Adsorption wird in einem Temperaturbereich von 0-150 °C, bevorzugt von 30-100 °C, besonders bevorzugt von 60-80 °C und bei Drücken von 0,05-5 bar, bevorzugt bei 0,2-4 bar, besonders bevorzugt bei 1-3,5 bar durchgeführt.

Das erfindungsgemäß gereinigte Ammoniak kann somit problemlos in verschiedene HCN- oder andere Herstell-Prozesse als Edukt zum Einsatz kommen. Beispielsweise ist die Umsetzung von Ammoniak mit Methanol zu HCN, in EP 0941984 dargelegt. Des Weiteren kann HCN aus Ammoniak und Methan gemäß dem BMA- oder Andrussow-Verfahren erhalten werden, wobei diese Verfahren in Ullmann's Encyclopedia of Industrial Chemistry 5. Auflage auf CD-ROM, Stichwort "Inorganic Cyano Compounds" beschrieben sind. Ebenso kann Ammoniak z.B. in einen Ammoxidationsprozess, wie beispielsweise die großtechnische Synthese von Acrylnitril aus Ammoniak, Sauerstoff und Propen zurückgeführt werden. Die Acrylnitrilsynthese ist z.B. unter dem Stichwort Sohio-Prozess in Industrial Organic Chemistry von K. Weisermehl und H.-J. Arpe auf Seite 307 ff. beschrieben.

Die folgenden Beispiele sollen das erfindungsgemäße Verfahren verdeutlichen aber in keinster Weise einschränken.

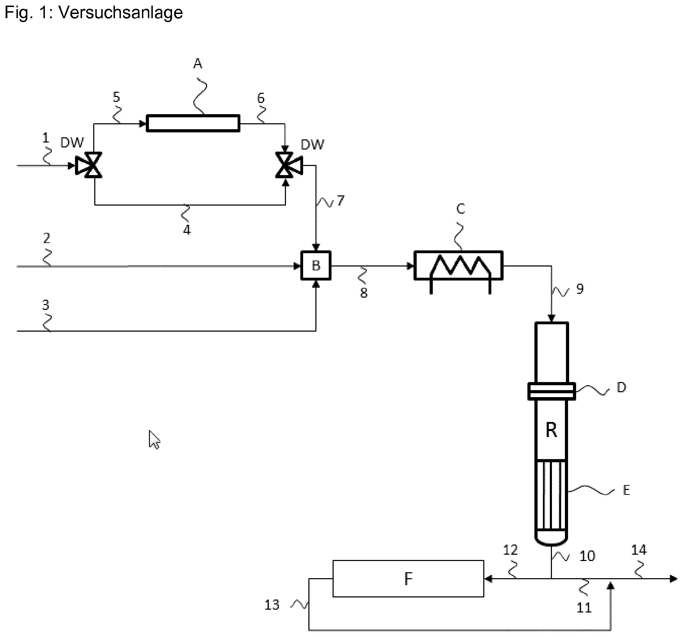

Die Beispiele und Vergleichsbeispiele wurden in einer Anlage wie in Fig. 1 gezeigt durchgeführt. Über die drei Dosierungsleitungen 1, 2 und 3 werden Ammoniak, Luft und Methan in die Versuchsanlage eingeleitet. Ammoniak kann mittels der beiden Dreiwegeventile (DW) entweder direkt oder über ein Adsorberbett (A), das mit Aktivkohle gefüllt ist, dem statischen Mischer (B) zugeführt werden. Das Adsorberbett ist beheizbar. Die Edukte werden im statischen Mischer gemischt und dann über Leitung 8 dem Vorwärmer (C) zugeführt, auf die gewünschte Temperatur vorgeheizt und über Leitung 9 in den Reaktor (R) eingeleitet. Dieser ist mit einem Katalysatornetz (D) und einem Luftkühler (E) ausgestattet, in letzterem wird das Produktgasgemisch auf eine gewünschte Temperatur abgekühlt. Dieses wird dann partiell über Leitung 12 einer Online-Analytik bzw. über Leitung 11, da im Versuchsfall keine Lagerung des entstehenden HCN vorgesehen ist, einer Verbrennung zugeführt.

### Bezeichnungsliste:

1: Ammoniak-Zuleitung
2: Luft-Zuleitung
3: Methan-Zuleitung
4: Umgehungsleitung Adsorberbett
5: Zuleitung Adsorber
6: Ausleitung Adsorber
7: Zuleitung statischer Mischer
8: Zuleitung Vorwärmer
9: Zuleitung Andrussow-Reaktor
10: Ausleitung Andrussow-Reaktor
11: Partielle Ausleitung Produktgemisch
12: Zuleitung Online-Analytik
13: Ausleitung Online-Analytik
14: Gesamte Ausleitung Produktgemisch
A: Adsorber-Bett
B: Statischer Mischer
C: Vorwärmer
D: Katalysatornetz
E: Luft Kühler
F: Online-Analytik
R: Reaktor

### Vergleichsbeispiel 1-2, Beispiel 1:

Diese werden in einer Anlage analog Fig. 1 durchgeführt. Im Vergleichsbeispiel 1 wird reines Ammoniak eingesetzt, im Vergleichsbeispiel 2 wird diesem 34 Gew% Reaktionsammoniak aus der HIBA-Alkoholyse und einen Katalysator zugesetzt. In beiden Fällen wird der Ammoniak-Feed nicht über das Adsorberbett geleitet. Im erfindungsgemäßen Beispiel 1 wird Vergleichsbeispiel 2 wiederholt, allerdings wird der gesamte Ammoniak-Feed über das Adsorberbett, das mit Aktivkohle Hydrafin CC 12x40 gefüllt ist, geleitet. Die Ergebnisse sind in Tab. 1 für Vergleichsbeispiel 1 und Beispiel 1 nach einer TOS (time on stream) von 14 d und für Vergleichsbeispiel 2 nach einer TOS von 1 h gezeigt. Ebenso sind die Amin-Verunreinigungen des Reaktionsammoniaks vor Einleitung in das Adsorberbett dargestellt.

**Tab. 1: Einfluss Aktivkohle**

| | | Vergleichsbeispiel 1 | Vergleichsbeispiel 2 | Beispiel 1 |
|---|---|---|---|---|
| Ammoniak (99,9 %) / g/min | | 2,506 | 0,906 | 0,799 |
| Reaktionsammoniak / g/min | | 0 | 1,600 | 1,600 |
| NH₃ (Gew.%) | 92,0 | | | |
| MeOH (Gew%) | 1,5 | | | |
| TMA (Gew%) | 5,5 | | | |
| DME (Gew.%) | 1,0 | | | |
| Methan (Linde 3.8) (g/min) | | 2,25 | 2,096 | 1,95 |
| Luft (g/min) | | 21,82 | 19,74 | 19,90 |
| N Gesamt / C Gesamt (mol/mol) | | 1,049 | 1,034 | 1,032 |
| NH₃/CH₄ (L/L) | | 1,049 | 1,069 | 1,080 |
| Luft/(NH₃+CH₄) (L/L) | | 2,63 | 2,51 | 2,72 |
| Ausbeute HCN/NH₃ (mol%) | | 63 | 13 | 65 |

Die Ausbeute an HCN ist mit aktivkohle-gereinigten Ammoniak-Feed enthaltend Reaktionsammoniak gegenüber nicht gereinigtem signifikant erhöht und mit reinem Ammoniak vergleichbar.

### Beispiel 2-4:

Beispiel 1 wurde wiederholt und die Adsorption bei verschiedenen Temperaturen des Adsorberbettes durchgeführt. Dabei wurde Alcarbon PH 55x8C der Fa. Donau Carbon als Aktivkohle eingesetzt. Die Ergebnisse sind in Tab 2. dargestellt

**Tab. 2: Temperaturabhängigkeit**

| | Beispiel 2 | Beispiel 3 | Beispiel 4 |
|---|---|---|---|
| Temperatur Adsorberbett (°C) | 20°C | 70°C | 100°C |
| NH₃ (Gew%) | 95,783 | 95,084 | 95,084 |
| TMA (Gew%) | 2,097 | 2,512 | 2,512 |
| DME (Gew%) | 0,792 | 0,753 | 0,753 |
| MeOH (Gew%) | 1,328 | 1,65 | 1,65 |
| Ausbeute HCN/NH₃ mol% | 63,26 | 62,57 | 62,51 |

Die Behandlung mit Aktivkohle bringt über einen weiten Temperaturbereich gute Ergebnisse hinsichtlich Ausbeute an HCN.

## Patentansprüche

1. Verfahren zur Herstellung von alpha-Hydroxycarbonsäureestern ausgehend von Blausäure **dadurch gekennzeichnet, dass** im Alkoholyseschritt des entsprechenden alpha-Hydroxycarbonsäureamids entstehendes Ammoniak nach einem Reinigungsschritt mit Aktivkohle noch enthaltend mindestens ein Alkylamin in den Blausäureherstellprozess über BMA- oder Andrussow-Prozess oder einen Ammonoxidations-Prozess zur Herstellung von Acrylnitril rezykliert wird.

2. Verfahren gemäß Anspruch 1 **dadurch gekennzeichnet, dass** die Konzentration der Gesamtmenge der Alkylamine bezogen auf Ammoniak 1-100000 ppm beträgt.

3. Verfahren gemäß Anspruch 1 **dadurch gekennzeichnet, dass** es sich beim alpha-Hydroxycarbonsäureester um Hydroxyisobuttersäuremethylester handelt.

4. Verfahren gemäß Anspruch 1 **dadurch gekennzeichnet, dass** es sich beim Alkylamin um Trimethylamin handelt.

5. Verfahren gemäß Anspruch 1 **dadurch gekennzeichnet, dass** die Blausäureherstellung nach Andrussow erfolgt.

6. Verfahren gemäß Anspruch 1 **dadurch gekennzeichnet, dass** die Reinigung des Ammoniaks adsorptiv durch Überleitung über einen Feststoff erfolgt.

7. Verfahren gemäß Anspruch 1 **dadurch gekennzeichnet, dass** die Reinigung des Ammoniaks in einem kontinuierlich betriebenen Adsorberbett erfolgt.

8. Verfahren gemäß Anspruch 1, **dadurch gekennzeichnet, dass** die Alkoholyse Reaktion des Hydroxycarbonsäureamids in flüssiger Phase oder in der Gasphase erfolgt

9. Verfahren gemäß Anspruch 1, **dadurch gekennzeichnet, dass** die Reinigung in einem Temperaturbereich von 0 °C bis 150 °C erfolgt.

10. Verfahren gemäß Anspruch 1, **dadurch gekennzeichnet, dass** die Reinigung in einem Druckbereich von 0,05 bis 5 bar erfolgt.

11. Verfahren gemäß Anspruch 1 **dadurch gekennzeichnet, dass** man
a) Eduktströme umfassend ein alpha-Hydroxycarbonsäureamid und einen Alkohol in einen Reaktor, der einen Katalysator enthält, einspeist,
b) diese Reaktionsmischung im Druckreaktor bei einem Druck im Bereich von 0,1-100 bar miteinander umsetzt,
c) die aus b) entstehende Produktmischung an Alkohol und Ammoniak abreichert,
d) Alkohol und Ammoniak enthaltend mindestens Trimethylamin trennt und
e) Ammoniak enthaltend mindestens Trimethylamin vor der Dosierung in das Blausäureherstellverfahren mittels Aktivkohle reinigt.

## Claims

1. Process for preparing alpha-hydroxycarboxylic esters proceeding from hydrogen cyanide, **characterized in that** ammonia formed in the step of alcoholysis of the corresponding alpha-hydroxycarboxamide, after a purification step with activated carbon, is recycled still comprising at least one alkylamine into the hydrogen cyanide preparation process via the BMA or Andrussow process or an ammoxidation process.

2. Process according to Claim 1, **characterized in that** the concentration of the total amount of the alkylamines based on ammonia is 1-100 000 ppm.

3. Process according to Claim 1, **characterized in that** the alpha-hydroxycarboxylic ester is methyl hydroxyisobutyrate.

4. Process according to Claim 1, **characterized in that** the alkylamine is trimethylamine.

5. Process according to Claim 1, **characterized in that** the hydrogen cyanide preparation is according to Andrussow.

6. Process according to Claim 1, **characterized in that** the ammonia is purified by adsorptive means by passage through a solid.

7. Process according to Claim 1, **characterized in that** the ammonia is purified in a continuously operated adsorber bed.

8. Process according to Claim 1, **characterized in that** the alcoholysis reaction of the hydroxycarboxamide is effected in the liquid phase or in the gas phase.

9. Process according to Claim 1, **characterized in that** the purification is effected within a temperature range from 0°C to 150°C.

10. Process according to Claim 1, **characterized in that** the purification is effected within a pressure range from 0.05 to 5 bar.

11. Process according to Claim 1, **characterized in that**
a) reactant streams comprising an alpha-hydroxycarboxamide and an alcohol are fed into a reactor containing a catalyst,
b) this reaction mixture is converted in the pressure reactor at a pressure in the range of 0.1-100 bar,
c) the product mixture of alcohol and ammonia that arises from b) is depleted,
d) alcohol and ammonia containing at least trimethylamine is separated and
e) ammonia containing at least trimethylamine is purified by means of activated carbon before being metered into the hydrogen cyanide preparation process.

## Revendications

1. Procédé pour la production d'esters d'acides alpha-hydroxycarboxyliques à partir d'acide cyanhydrique, **caractérisé en ce qu'**on recycle de l'ammoniac formé dans l'étape d'alcoolyse de l'alpha-hydroxycarboxamide correspondant, après une étape de purification avec du charbon actif contenant encore au moins une alkylamine, dans le processus de production d'acide cyanhydrique par le procédé BMA ou le procédé d'Andrussow ou un processus d'ammonoxydation pour la production d'acrylonitrile.

2. Procédé selon la revendication 1, **caractérisé en ce que** la concentration de la quantité totale des alkylamines par rapport à l'ammoniac est de 1 - 100 000 ppm.

3. Procédé selon la revendication 1, **caractérisé en ce que** pour ce qui est de l'ester d'acide alpha-hydroxycarboxylique il s'agit de l'ester méthylique d'acide hydroxyisobutyrique.

4. Procédé selon la revendication 1, **caractérisé en ce que** pour ce qui est de l'alkylamine il s'agit de la triméthylamine.

5. Procédé selon la revendication 1, **caractérisé en ce que** la production d'acide cyanhydrique s'effectue selon Andrussow.

6. Procédé selon la revendication 1, **caractérisé en ce que** la purification de l'ammoniac s'effectue par adsorption par passage sur une matière solide.

7. Procédé selon la revendication 1, **caractérisé en ce que** la purification de l'ammoniac s'effectue dans un lit d'adsorbant utilisé en continu.

8. Procédé selon la revendication 1, **caractérisé en ce que** la réaction d'alcoolyse de l'hydroxycarboxamide s'effectue en phase liquide ou dans la phase gazeuse.

9. Procédé selon la revendication 1, **caractérisé en ce que** la purification s'effectue dans une plage de température de 0 °C à 150 °C.

10. Procédé selon la revendication 1, **caractérisé en ce que** la purification s'effectue dans une plage de pression de 0,05 à 5 bars.

11. Procédé selon la revendication 1, **caractérisé en ce que**
a) on introduit des courants de départ comprenant un alpha-hydroxycarboxamide et un alcool dans un réacteur qui contient un catalyseur,
b) on fait réagir ce mélange réactionnel dans un réacteur à pression, sous une pression dans la plage de 0,1-100 bars,
c) on appauvrit en alcool et ammoniac le mélange de produits résultant de b),
d) on sépare l'alcool et l'ammoniac contenant au moins de la triméthylamine et
e) on purifie au moyen de charbon actif l'ammoniac contenant au moins de la triméthylamine, avant l'introduction dans le procédé de production d'acide cyanhydrique.
